# EUROPÄISCHE PATENTSCHRIFT

(11) **EP 1 937 253 B1**
(45) Veröffentlichungstag und Bekanntmachung des Hinweises auf die Patenterteilung: **13.06.2012**
(21) Anmeldenummer: 06806014.4
(22) Anmeldetag: 04.10.2006
(51) Int. Cl.: A61K 31/40, A61K 31/366, A61K 31/22, A61K 31/44, A61K 31/60, A61K 9/20

(54) **KOMBINATIONSPRÄPARATE VON SALZEN DER O-ACETYLSALICYLSÄURE**
COMBINED PREPARATION OF O-ACETYLSALICYLIC ACID SALTS
ASSOCIATION MEDICAMENTEUSE DE SELS DE L'ACIDE O-ACETYLSALICYLIQUE

(30) Priorität: 15.10.2005 DE 102005049293
(43) Veröffentlichungstag der Anmeldung: 02.07.2008
(73) Patentinhaber: Bayer Pharma Aktiengesellschaft, 13353 Berlin (DE)
(72) Erfinder: LEDWOCH, Wolfram, 40764 Langenfeld (DE); HAASE, Claus, Gert, 45657 Recklinghausen (DE); FRANCKOWIAK, Gerhard, 42115 Wuppertal (DE); WAGNER, Heike, 69117 Heidelberg (DE); HAYAUCHI, Yutaka, 51373 Leverkusen (DE)
(86) Internationale Anmeldenummer: PCT/EP2006/009573
(87) Internationale Veröffentlichungsnummer: WO 2007/045353

(56) Entgegenhaltungen:
- WO-A-02/05782
- WO-A-03/059323
- WO-A-2004/080488
- DE-A1- 10 351 648
- GB-A- 2 051 573

## Beschreibung

Die vorliegende Erfindung betrifft eine fixe Kombination enthaltend ein Salz von o-Acetylsalicylsäure mit einer basischen Aminosäure als Komponente A und einem HMG-CoA-Reduktase-Inhibitor als Komponente B, ein Arzneimittel enthaltend diese Kombination und ein Verfahren zu dessen Herstellung.

Die analgetische Wirkung von o-Acetylsalicylsäure wird seit langem therapeutisch genutzt. So wird o-Acetylsalicylsäure als Analgetikum, Antipyretikum, Antirheumatikum, sowie als nichtsteroidaler entzündungshemmender Wirkstoff beispielsweise zur Behandlung von Arthritis, Neuralgien und Myalgien eingesetzt. Des weiteren wird o-Acetylsalicylsäure aber auch zur sekundären Prävention (Antiplatelet Trialists' Collaboration: Collaborative overview of randomised trials of antiplatelet therapy. I: Prevention of death, myocardial infarction, and stroke by prolonged antiplatelet therapy in various categories of patients. Brit. Med. J. 1994, 308, 81-106) und zur primären Prävention von Herzkreislauferkrankungen (U.S. Preventive Services Task Force: Aspirin for the primary prevention of cardiovascular events: recommendation and rationale. Ann. Intern. Med. 2002, 136, 157-160) eingesetzt.

Bekannte Salze der o-Acetylsalicylsäure (ASS) sind unter anderem Salze von o-Acetylsalicylsäure mit basischen Aminosäuren. Durch die orale Gabe beispielsweise des Lysinsalzes der o-Acetylsalicylsäure (D,L-Lysin-o-Acetylsalicylat) kann eine Anflutung des Wirkstoffs erreicht werden, die beinahe der Blutspiegelkurve einer Bolusinjektion entspricht. Dies ist in der Literatur beschrieben [Ch. Raschka, H. J. Koch, Perfusion 6 (2000), 13. Jahrgang, Verlag PERFUSION, Nürnberg]. Der Wirkstoff löst sich außergewöhnlich schnell auf und wird sofort vom Körper resorbiert.

Therapeutisch wird beispielsweise das Salz der o-Acetylsalicylsäure (ASS) mit der Aminosäure Lysin eingesetzt. Bei dem gängigsten Medikament mit D,L-Lysin-o-Acetylsalicylat handelt es sich um eine Darreichungsform zur parenteralen Applikation enthaltend zusätzlich Glycin. Es ist unter dem Namen Aspisol® (bis Mitte 2005) im Handel. Das Glycin wird dem ASS-Lysinat fest zugegeben, so dass ein Gemenge aus D,L-Lysin-o-Acetylsalicylat und Glycin vorliegt.

In der WO 02/005782 und der WO 03/059323 werden Salze von o-Acetylsalicylsäure mit basischen Aminosäuren beschrieben, die eine erhöhte Stabilität aufweisen. Die Salze werden nach einem besonderen Verfahren hergestellt und zeichnen sich bei einer mit einem Malvern 2600D-Gerät unter Standardbedingungen gemessenen Korngrößenverteilung durch eine mittlere Korngröße oberhalb einer Korngröße von 160 µm und einen Anteil von mehr als 60 % der Teilchen mit einer Korngröße in einem Bereich von 100 bis 200 µm aus. Sie können einen gewissen Gehalt an zugegebenem Glycin aufweisen, der aber keinen Einfluss auf die Eigenschaften des o-Acetylsalicylats hat, insbesondere auf dessen Stabilität.

HMG-CoA-Reduktase-Inhibitoren, zu denen die Klasse der Statine gehört, sind dem Fachmann als Lipidspiegelsenker gut bekannt. Die Wirkung der Statine in der primären Prävention von Herzkreislauferkrankungen durch Senkung des Cholesterinspiegels (Heart Protection Study of cholesterin lowering with simvastatin, LANCET 360, 2002, 7-22) und auch in der sekundären Prävention (The Scandinavian Simvastatin Survival Study, The Lancet 344, 1994, 1383-1389) ist am Beispiel des Simvastatins beschrieben. WO 99/47123 offenbart die Kombination von o-Acetylsalicylsäure (ASS) mit Statinen, insbesondere Pravastatin, Lovastatin, Simvastatin, Atorvastatin, Fluvastatin oder Cerivastatin, sowie die Verwendung dieser Kombination zur Senkung des Cholesterinspiegels im Blut und zur Prävention und Behandlung von Herzkreislauferkrankungen.

WO 99/47123 geht von der chemischen Inkompatibilität zwischen o-Acetylsalicylsäure und Statinen aus und beschreibt eine spezielle pharmazeutische Formulierung enthaltend ASS und einem Statin, insbesondere Pravastatin, die die Interaktion zwischen o-Acetylsalicylsäure und Statin reduziert, indem beide Komponenten getrennt voneinander beispielsweise in unterschiedlichen Schichten vorliegen.

Die geringe Stabilität der o-Acetylsalicylsäure und ihrer Salze ist auf eine dem Fachmann bekannte Freisetzung von Salicylsäure zurückzuführen. Die Anwesenheit freier Salicylsäure in pharmazeutischen Zubereitungen ist jedoch unerwünscht und deshalb auf einen geringen, akzeptablen Wert zu begrenzen.

Aufgabe der vorliegenden Erfindung ist die Bereitstellung einer ausreichend stabilen und einfach herzustellenden pharmazeutischen Darreichungsform, die geeignet ist, o-Acetylsalicylsäure und Statin freizusetzen.

Überraschenderweise wurde nun gefunden, daß die erfindungsgemäßen Darreichungsformen enthaltend ein Salz von o-Acetylsalicylsäure mit einer basischen Aminosäure als Komponente A und Statine als Komponente B ausreichend stabil und leicht herzustellen sind.

Gegenstand der vorliegenden Erfindung ist eine fixe Kombination enthaltend ein Salz von o-Acetylsalicylsäure mit einer basischen Aminosäure als Komponente A und einen HMG-CoA-Reduktase-Inhibitor als Komponente B.

Die erfindungsgemäß als Komponente des Salzes von o-Acetylsalicylsäure geeignete basische Aminosäure kann in der L- oder in der D-Konfiguration auftreten oder auch als Gemisch von D-und L-Form. Der Begriff "Aminosäure" bezeichnet erfingdungsgemäß insbesondere die in der Natur vorkommenden α-Aminosäuren, umfasst darüber hinaus aber auch deren Homologe, Isomere und Derivate. Als Beispiel für Isomere können Enantiomere genannt werden. Derivate können beispielsweise mit Schutzgruppen versehene Aminosäuren sein. Als typische Beispiele für basische Aminosäuren seien genannt: Lysin, Arginin, Ornithin, Diaminobuttersäure. Das Salz der o-Acetylsalicylsäure mit Lysin ist besonders gut geeignet. Dabei kann das Lysin in der D- oder L-Form, oder als Gemisch von D- und L-Form vorliegen. Schutzgruppen für Aminosäuren sind dem Fachmann bekannt und sind in T.W. Greene, P.G. Wuts, Protective Groups in Organic Synthesis, 3rd ed., JohnWiley, New York, 1999 beschrieben.

Bezüglich Einzelheiten zu HMG-CoA-Reduktase-Inhibitoren - die Abkürzung "HMG-CoA" steht hierbei für "3-Hydroxy-3-methylglutaryl-Coenzym A" - und insbesondere zu den Statinen wird verwiesen auf die Abhandlungen in Drugs of the Future 1994, 19(6), Seiten 537 - 541 sowie 1995, 20(6), Seite 611 sowie 1996, 21(6), Seite 642. Eine weitere Übersicht über HMG-CoA-Reduktase-Inhibitoren ist in Pharmazie in unserer Zeit, 28. Jahrgang, Nr. 3, Seiten 147-152 (1999) enthalten.

EMG-CoA-Reduktase-Inhibitoren als Komponente B stehen im Rahmen der Erfindung im Allgemeinen für alle im Stand der Technik unter diesem Begriff aufgeführten Stoffklassen. Bevorzugt sind unter diesem Begriff Statine, wie sie beispielsweise in EP 247 633, US 5 006 530, EP 33 538, US 4 346 227, EP 22 478, EP 491 226, EP 325 130 oder EP 114 027 beschrieben sind.

Bevorzugt aus der Gruppe der Statine sind Pravastatin, Lovastatin (MEVACOR®; US 4 231 938), Simvastatin (ZOCOR®; US 4 444 784), Atorvastatin (LIPITOR®; US 5 273 995), Fluvastatin (LESCOL®; US 5 354 772), Itavastatin ("NK-104", siehe EP 0304 063), Pitavastatin, Rosuvastatin sowie ZD-4522 (siehe EP 521 471; systematischer Name: (+)-(3R,5S)-Bis(7-(4-(4-fluorphenyl)-6-isopropyl-2-(N-methyl-N-methansulfonylamino)pyrimidin-5-yl)-3,5-dihydroxy-6(E)-heptensäure, Calciumsalz) oder Cerivastatin (US 5 177 080).

Besonders bevorzugt sind Atorvastatin, Pravastatin, Lovastatin und Simvastatin. Ganz besonders bevorzugt ist Simvastatin.

Statine können in Form ihrer Ester oder Lactone oder als Carbonsäure bzw. Salze der Carbonsäure vorliegen. Bei Cerivastatin wird besonders bevorzugt das Natriumsalz (Cerivastatin-Natrium) eingesetzt. Herstellverfahren zu den HMG-CoA-Reduktase-Inhibitoren sind dem Fachmann bekannt.

### Arzneimittel:

Überraschenderweise wurde gefunden, dass es möglich ist, die beiden Komponenten A und B in Form einer fixen Kombination zu einer stabilen Formulierung zu verarbeiten, in der beide Komponenten A und B zusammen in einer gemeinsamen Matrix in derselben Schicht vorliegen.

Unter "fixer Kombination" im Sinne dieser Erfindung werden solche Arzneiformen verstanden, in der die Komponenten gemeinsam in einem festgelegten Mengenverhältnis vorliegen. Eine solche fixe Kombination kann beispielsweise als Tablette, Filmtablette, Dragee, Granulat, Kapsel oder Pulver realisiert werden.

Die Darreichungsform enthaltend die erfindungsgemäße fixe Kombination der Komponenten A und B kann gegebenenfalls unter Zusatz geeigneter Hilfsstoffe durch Herstellung beispielsweise einer Mischung beider Komponenten und direkter Abfüllung beispielsweise in Sachets oder Kapseln oder Formulierung zu Tabletten erhalten werden.

Pharmazeutische Hilfsstoffe, wie sie dem Fachmann geläufig sind, sind beispielsweise auch in folgendem Handbuch beschrieben: "Handbook of Pharmaceutical Excipients", Wade, A. & Weller, P.J., American Pharmaceutical Association, Washington, 2nd edition 1994.

Hilfsstoffe sind solche, die pharmazeutisch akzeptiert und physiologisch unbedenklich sind, beispielsweise Füllstoffe, Sprengmittel und Gleitmittel. Beispiele für die genannten Hilfsstofffgruppen können sein als Füllstoffe Cellulosederivate (z.B. mikrokristalline Cellulose), native und modifizierte Stärken (z.B. Kartoffelstärke), Zucker (z.B. Lactose), Zuckeralkohole (z.B. Mannit, Sorbit), anorganische Füllstoffe (z.B. Calciumphosphat, Magnesiumoxid) und Puffersubstanzen; als Sprengmittel Stärkederivate (z.B. quervernetzte Natriumcarboxy-methylstärke, Natriumstärkeglykolat), Cellulosederivate (z.B. quervernetzte Natriumcarboxymethylcellulose) und quervernetztes Polyvinylpyrrolidon; als Gleitmittel (hier verstanden als Oberbegriff für Fließregulierungsmittel/Schmiermittel/Formentrennmittel): Magnesiumstearat, Calciumstearat, Stearinsäure, Talkum und hochdisperses Siliciumdioxid.

Um eine gleichmäßige Verteilung der beiden Substanzen in der Mischung zu gewährleisten, werden zunächst die beiden Komponenten A und B miteinander vermischt und hierzu die Hilfsstoffe in Etappen unter gemischt. Während der Herstellung und Lagerung ist gegebenenfalls auf angemessenen Feuchteschutz zu achten.

Gegenstand der vorliegenden Erfindung ist auch eine Weiterverarbeitung der bereits beschriebenen Wirkstoff/Hilfsstoff-Mischung zu einem Granulat. Zur Granulation eignen sich erfindungsgemäß die bekannten herkömmlichen Verfahren. Bevorzugtes Granulationsverfahren ist die Trockengranulation, insbesondere die Walzenkompaktierung. Zuzüglich zu den bereits genannten Hilfsstoffen kann der Einsatz von Bindemitteln beispielsweise Zuckern, Zuckeralkoholen, Stärken, Cellulosederivaten, Alginaten, Pektinen, Polyethylenglykolen und Polyvinylpyrrolidon erfolgen. Der Zusatz von Antistatika ist möglich. Hier bieten sich beispielsweise Polyethylenglykole, Natriumlaurylsulfat, hochdisperses Siliciumdioxid/Aluminiumoxid oder Dicalciumphosphat an. Das produzierte Granulat kann direkt abgefüllt werden beispielsweise in Sachets oder Kapseln oder kann zu Tabletten formuliert werden. Auf ausreichenden Feuchteschutz während der Herstellung und Lagerung ist gegebenenfalls zu achten.

Darüber hinaus kann die Herstellung der erfindungsgemäßen fixen Kombination auch durch die Produktion zweier voneinander unabhängiger Mischungen oder Granulate enthaltend die Komponenten A und B und gegebenenfalls weitere Hilfsstoffe erfolgen, die in einem zusätzlichen Prozessschritt vereint und anschließend abgefüllt werden beispielsweise in Sachets oder Kapseln oder zur Tablette formuliert werden. Jeder einzelne Herstellungsschritt kann durch den Zusatz geeigneter Hilfsstoffe gekennzeichnet sein. Das bevorzugte Granulationsverfahren für Komponente A ist in diesem Fall ein Trockengranulationsverfahren, insbesondere die Walzenkompaktierung. Komponente B kann sowohl trocken als auch feucht granuliert werden. Bei der Feuchtgranulation kann als Lösungsmittel Wasser, ein geeignetes organisches Lösungsmittel wie beispielsweise Ethanol, Aceton oder Isopropanol, oder eine Mischung aus Wasser mit einem geeigneten organischen Lösungsmittel eingesetzt werden. Der Zusatz von Hydrophilisierungsmitteln in Form von Tensiden beispielsweise Natriumlaurylsulfat, Polysorbate ist möglich. Bei Einsatz eines Feuchtgranulationsverfahrens ist auf eine geringe Restfeuchte des Granulates zu achten. Nach der Vereinigung der Granulate der Komponenten A und B ist gegebenenfalls bei den folgenden Prozessschritten (Fertigung und Lagerung) ein genügender Feuchteschutz zu gewährleisten.

Ebenfalls Gegenstand der vorliegenden Erfindung ist die Herstellung einer fixen Kombination unter Einsatz der Schmelzextrusion, einem Prozess, der ebenfalls Standardverfahren folgt. Hierbei kann Komponente A in Form der bereits beschrieben Wirkstoff/Hilfsstoffmischung oder dessen Granulat eingesetzt werden, während Komponente B zunächst zu einem Schmelzextrudat verarbeitet wird. Für den Prozess der Schmelzextrusion ist der Einsatz mindestens eines Polymers, Weichmachers und/oder Trägers notwendig. Geeignete Polymere sind beispielsweise Hydroxypropylcellulose und Polyvinylpyrrolidon. Als Weichmacher kommen beispielsweise Triethycitrat, Benzoesäure und Bersteinsäure in Frage. Unter dem Begriff Trägersubstanzen werden hier Hilfsstoffe wie beispielsweise Zuckeralkohole, insbesondere Mannit, mikrokristalline Cellulose und quervernetzte Natriumcarboxymethylcellulose verstanden. Das erhaltene Schmelzextrudat enthaltend Komponente B wird nach seiner Herstellung zerkleinert, vorzugsweise auf eine Korngröße von kleiner 0,315 mm. Nach Zusammengeben des Schmelzextrudats enthaltend Komponente B und der Wirkstoff/Hilfsstoffmischung oder Granulatmischung enthaltend die Komponente A kann eine direkte Abfüllung als Granulat beispielsweise in Sachets oder Kapseln oder eine Kompaktierung zu Tabletten erfolgen. Ein Zusatz von Schmiermittel ist unter diesen Bedingungen zu empfehlen. Nach Vereinigung der Wirkstoff-/Hilfsstoffzubereitungen der Komponenten A und B in Form des Mischungsschrittes ist gegebenenfalls auf ausreichenden Feuchteschutz zu achten.

Darüber hinaus kann die Herstellung der erfindungsgemäßen fixen Kombination enthaltend Komponente A und B auch durch die Herstellung zweier voneinander unabhängiger Schmelzextrudate erfolgen, die in einem zusätzlichen Prozessschritt vereint und anschließend abgefüllt beispielsweise in Sachets oder Kapseln oder zu Tabletten formuliert werden. Jeder einzelne Herstellungsschritt kann durch den Zusatz geeigneter Hilfsstoffe gekennzeichnet sein. Nach erfolgter Schmelzextrusion schließt sich eine Zerkleinerung der Schmelzextrudate, vorzugsweise auf eine Korngröße von kleiner 0,315 mm, an. Die so erhaltenen Granulate werden gemischt. Das Mischungsgranulat kann direkt verpackt werden, beispielsweise in Sachets oder Kapseln, oder zu Tabletten verpresst werden. Hilfsstoffe wie bereits beschrieben können eingesetzt werden. Unabhängig von der Endformulierung ist gegebenenfalls angemessener Feuchteschutz zu gewährleisten.

Darüber hinaus ist die Herstellung einer fixen Kombination enthaltend die Komponente A und B auch durch die gemeinsame Schmelzextrusion beider Wirkstoffe in einer Wirkstoff/Hilfsstoff-Mischung Gegenstand dieser Erfindung. Nach erfolgter Schmelzextrusion schließt sich eine Zerkleinerung des Schmelzextrudats, vorzugsweise auf eine Korngröße von kleiner 0,315 mm, an. Das so erhaltene Granulat kann direkt verpackt werden, beispielsweise in Sachets oder Kapseln, oder zu Tabletten verpresst werden. Hilfsstoffe wie bereits beschrieben können eingesetzt werden. Unabhängig von der Endformulierung ist gegebenenfalls angemessener Feuchteschutz zu garantieren.

Die hergestellten, die erfindungsgemäße fixe Kombination enthaltenden Darreichungsformen können im letzten Herstellungsschritt mit einem Überzug versehen werden. Überzüge im Sinne dieser Erfindung können Zuckerüberzüge und insbesondere Filmüberzüge sein. Der Prozess folgt allgemeinen Standardverfahren und macht den Zusatz weiterer Hilfsstoffe erforderlich. Für die Filmüberzüge werden Filmbildner wie beispielsweise modifizierte Cellulosen, Polymethacrylate, Polyvinylpyrrolidon und Polyvinylacetatphthalat, Weichmacher wie beispielsweise Polyethylenglykole, Triacetin, Glycerin, Dibutylphthalat, Farbstoffe/Pigmente wie beispielsweise Titandioxid, Eisenoxid und/oder Trennmittel wie beispielsweise Talkum, hochdisperses Siliciumdioxid, Silicagel, Magnesiumstearat, Glycerolmonostearat verwendet. Das Überziehen erfolgt durch Aufsprühen einer Suspension, bevorzugt auf wässriger Basis. Ein Austausch des Wassers durch ein geeignetes organisches Lösungsmittel ist möglich.

Zur Ausführung als Kapsel eignen sich sowohl die üblichen Hartgelatine-Steckkapseln als auch Steckkapseln aus HPMC (Methylhydroxypropylcellulose). Die HPMC-Steckkapseln lassen sich direkt oder nach Trocknung einsetzen. Überraschenderweise sind sowohl die erfindungsgemäße Kombination als auch die Einzelkomponenten A und B nicht nur in den HPMC-Steckkapseln sondern auch in den Hartgelatinesteckkapseln stabil. Dies demonstriert erneut die Stabilität der erfindungsgemäßen Kombination und Arzneimittel. Weiterhin sind auch Steckkapseln aus anderen Polymeren (z.B. Stärke, Cellulose, Hydroxypropylcellulose, Hydroxypropylcelluloselactat, Hydroxypropylcelluloseglycolid und Hydroxyethylhydroxypropylcellulose) geeignet.

Insbesondere Komponente B kann kristallin oder amorph, gemahlen oder mikronisiert verarbeitet werden. Um die Resorption aus dem Magen-Darm-Trakt zu beschleunigen, ist die bevorzugte Partikelgröße der im Rahmen dieser Erfindung eingesetzten Komponente B eine mittlere Korngröße von X₅₀ ≤ 15 µm, bevorzugt X₅₀ ≤ 8 µm (siehe Figur 1: Beispiel für eine Partikelgrößenverteilung von Simvastatin mikronisiert).

Für die orale Applikation eignen sich bekannte, den Wirkstoffkomplex schnell und/oder modifiziert abgebende Applikationsformen, wie beispielsweise Tabletten (nichtüberzogene sowie überzogene Tabletten, magensaftresistente Überzüge, ODT (oral dissolving tablets), Brausetabletten, Kautabletten), Kapseln, Dragees, Granulate, Pellets oder Pulver.

Bei der erfindungsgemäßen fixen Kombination handelt es sich bevorzugt um eine die Wirkstoffe schnell-freisetzende Formulierung (siehe Figur 2: Beispiel für Freisetzungsprofile einer Tablette enthaltend die fixe Kombination aus D,L-Lysin-o-Acetylsalicylat und Simvastatin). Unter "schnell-freisetzender Formulierung" soll hier eine solche Formulierung (z.B. Tablette, Kapsel, Sachet) verstanden werden, die mindetstens 80% ihrer enthaltenden Wirkstoffdosen innerhalb von 30 Minuten freisetzt (Blattrührer-Apparatur nach USP; UV-Detektion).

Die fixe Kombination im Sinne dieser Erfindung kann bis zu dreimal täglich appliziert werden, bevorzugt ist eine Kombination, die eine 1- bis 2-mal tägliche Gabe erlaubt.

Überraschenderweise zeichnet sich die erfindungsgemäße fixe Kombination enthaltend Komponente A und B durch eine hohe Stabilität aus. Eine signifikante Zersetzung von Komponente A und/oder B wird nicht beobachtet.

### Verwendung:

Die erfindungsgemäße Kombination kann zur Prävention von Herzkreislauferkrankungen eingesetzt werden. Unter Prävention wird sowohl eine primäre als auch sekundäre Prävention verstanden. Unter primärer Prävention versteht man in diesem Zusammenhang den Schutz von Patienten vor einer ersten Herzkreislauferkrankung, die eine Organschädigung zur Folge hat. Unter sekundärer Prävention versteht man in diesem Zusammenhang den Schutz von Patienten, die bereits eine Organschädigung in Folge einer Herzkreislauferkrankung erlitten haben, vor einer erneuten Herzkreislauferkrankung.

Bevorzugt wird die erfindungsgemäße Kombination zur Prävention von Herzkreislauferkrankungen bei Patienten, die ein erhöhtes Risiko besitzen, an einer Herzkreislauferkrankung zu erkranken, eingesetzt.

Zu dem hier erwähnten Patientenkreis mit erhöhtem Risiko gehören zum Beispiel Patienten mit Mikro- und Makroangiopathie, Patienten mit erhöhtem Blutdruck (Hypertonie), Patienten mit einem Risiko, an Bluthochdruck zu erkranken, Patienten mit renaler Dysfunktion wie zum Beispiel milder renaler Herzinsuffizienz (mild renal failure (MRF)), die einen erhöhten Kreatininspiegel im Blutplasma aufweisen, Patienten mit Diabetes, Patienten mit einem gestörten Glukosestoffwechsel (prä-diabetische Stoffwechsellage), Patienten mit einem erhöhten Body Mass Index (BMI), Patienten mit einem erhöhten Homocysteinspiegel, Patienten, die Angehörige ersten Grades haben, die an einer Herzkreislauferkrankung leiden oder gelitten haben, und Patienten, die Angehörige ersten Grades haben, die an Diabetes leiden oder gelitten haben. Ein erhöhter Kreatininspiegel liegt insbesondere bei einem Wert von über 1,5 mg/dl bei Männern und 1,4 mg/dl bei Frauen vor. Ein erhöhter BMI liegt insbesondere bei einem Wert von über 25 kg/m² vor. Insbesondere bei Patienten mit Hypertonie liegt ein erhöhtes Risiko vor bei einem Gesamtcholesterinspiegel von über 200 mg/dl und/oder einem LDL-Spiegel (low density lipoprotein) von über 160 mg/dl. Insbesondere bei Patienten mit Diabetes, prä-diabetischer Stoffwechsellage oder renaler Dysfunktion liegt ein erhöhtes Risiko vor bei einem Gesamtcholesterinspiegel von über 170 mg/dl und/oder einem LDL-Spiegel (low density lipoprotein) von über 120 mg/dl. Zu den Patienten mit erhöhtem Blutdruck gehören insbesondere Patienten mit nur geringfügig erhöhtem Blutdruck (120/85 mmHg bis 139/90 mmHg) und Patienten, die bei Vorhandensein eines erhöhten Blutdrucks den erhöhten Blutdruck insuffizient reduziert haben (> 145/95 mmHg). Ein erhöhtes Risiko liegt bei einem erhöhten Homocysteinspiegel von über 12 µmol/l im Blut vor.

Ebenfalls zu dem hier erwähnten Patientenkreis mit erhöhtem Risiko gehören Patienten, die bereits eine Organschädigung in Folge einer Herzkreislauferkrankung erlitten haben. Diese Patienten hatten bereits beispielsweise einen Schlaganfall, Angioplastie, Bypassoperation, Angina pectoris, Erkrankungen der Kranzgefäße des Herzens, Myokardinfarkt, Thrombose und/oder krankhafte Veränderungen der Gefäßwand.

Besonders bevorzugt gehören zu dem hier erwähnten Patientenkreis mit erhöhtem Risiko Patienten mit renaler Dysfunktion wie zum Beispiel milder renaler Herzinsuffizienz (mild renal failure (MRF)), die einen erhöhten Kreatininspiegel im Blutplasma aufweisen, Patienten mit nur geringfügig erhöhtem Blutdruck (120/85 mmHg bis 139/90 mmHg) und Patienten, die bei Vorhandensein eines erhöhten Blutdrucks den erhöhten Blutdruck insuffizient reduziert haben (> 145/95 mmHg).

Die erfindungsgemäße Kombination zeigt bei Patienten, die ein erhöhtes Risiko besitzen, an einer Herzkreislauferkrankung zu erkranken, ein unerwartetes breites und vielseitiges Wirkungsspektrum. Unter Herzkreislauferkrankungen werden Erkrankungen verstanden wie Mikro- und Makroangiopathie, Arteriosklerose, Schlaganfall, Angina pectoris, Erkrankungen der Kranzgefäße des Herzens, insbesondere der arteriellen Kranzgefäße, Herzversagen, Myokardinfarkt, krankhaften Veränderungen der Gefäßwand, Durchblutungsstörungen, Störungen der Mikrozirkulation, Fettstoffwechselstörungen wie Hyperlipidämie, Dyslipidämie, erhöhte Konzentration von Lipoproteinen im Serum und eventuell eine Verschiebung der Lipoproteinanteile, Hyperlipoproteinämie, Erhöhung sowohl des Serumcholesterins als auch der Serumtriglyceride kombiniert mit erhöhtem VLDL (very low density lipoprotein) und Erhöhung der Chylomikronen im Plasma, Störungen des Homocysteinspiegels, nicht-insulinabhängiger Diabetes mellitus (= Typ-2-Diabetes), Diabetes, Hyperglykämie, Stoffwechselstörungen wie Störung des Lipidmetabolismus, Defizienz der sauren Lipase, Speicherkrankheiten, insbesondere Fettspeicherkrankheiten, Phytosterolämie, Bluthochdruck, Fettsucht, Thrombosen, Pankreatitis, Verstopfung (Obstipation), Funktionsstörungen des Gehirns, zerebrovaskulärer Insuffizienz, zerebrale Durchblutungsstörungen, Apoplexie, transitorische ischämische Attacken (TIA), Demenz und Ohnmacht.

Von besonderem Interesse ist der Einsatz der erfindungsgemäßen Kombination beim sogenannten cardiac risk manangement, d.h. bei der Prophylaxe von Herzkreislauferkrankungen, die durch mehr als einen Risikofaktor beeinflusst bzw. verursacht werden wie z.B. Arteriosklerose, Erkrankungen der Kranzgefäße des Herzens, insbesondere der arteriellen Kranzgefäße, erhöhten Serumlipiden, Hypercholesterinämie, Hypertriglyzeridämie, Erhöhung sowohl des Serumcholesterins als auch der Serumtriglyceride kombiniert mit erhöhtem VLDL (very low density lipoprotein) und Erhöhung der Chylomikronen im Plasma und Syndrom X. Typische Risikofaktoren sind erhöhter Cholesterinspiegel, erniedrigter HDL-Spiegel, Rauchen, Glukoseintoleranz und Herzvergrößerung. Die Risikofaktoren können abhängig von Alter und Geschlecht des Patienten unterschiedlich sein.

Unter "Hyperlipidämie" soll ein erhöhter Plasmaspiegel eines oder mehrerer Serumlipide verstanden werden. In dieser Hinsicht ist besonders der LDL-Spiegel von Bedeutung. Als erhöhter Spiegel werden bei Patienten von über 45 Jahren Werte über 130 mg/dl und bei Patienten von unter 45 Jahren Werte über 160 mg/dl angesehen.

Unter "Dyslipidämie" soll hier entweder eine Hypertriglyceridämie oder eine Hypercholesterinämie, besonders aber eine gemischte Hyperlipidämie verstanden werden, d.h. ein Krankheitszustand mit erhöhtem Cholesterinspiegel (LDL und Gesamtcholesterin) und erhöhtem Triglyceridspiegel. Dies kann assoziiert sein mit einer Verminderung des HDL-(High-Density-Lipoprotein)-Cholesterins im Plasma oder einem gestörten HDL-C/LDL-C-Verhältnis.

Die erfindungsgemäße Kombination erweist sich als überraschend vorteilhaft bei der Prävention von Mikro- und Makroangiopathie, koronaren Herzerkrankungen, Herzinsuffizienz, Störung der Hirnleistung, Apoplexie, Durchblutungsstörungen, Störungen des Fettstoffwechsels, Bluthochdruck oder des Diabetes mellitus.

Der Effekt ist besonders stark bei Patienten mit Mikro- oder Makroangiopathie insbesondere in Folge von Bluthochdruck, Patienten mit Bluthochdruck, Patienten mit renaler Dysfunktion wie zum Beispiel milder renaler Herzinsuffizienz (mild renal failure (MRF)), Patienten, die einen erhöhten Kreatininspiegel im Blutplasma aufweisen, Patienten mit Diabetes und Patienten mit einem gestörten Glukosestoffwechsel (prä-diabetische Stoffwechsellage).

Insbesondere wurde gefunden, dass die erfindungsgemäße Kombination zu überraschenden Effekten bei Patienten führt, die ein erhöhtes Risiko besitzen, an einer Herzkreislauferkrankung zu erkranken, die aber einen normalen Cholesterinspiegel besitzen, die einen nur geringfügig erhöhten Blutdruck (120/85mmHg bis 139/90mmHg) besitzen oder die bei Vorhandensein eines erhöhten Blutdrucks den erhöhten Blutdruck insuffizient reduziert haben (RR > 145/95mmHg).

Bei Verwendung der erfindungsgemäßen Kombination wird bei der Wirkung ein nicht zu erwartender synergistischer Effekt beobachtet. Damit können die in der Kombination eingesetzten Mengen der Wirkstoffe im Vergleich zur Monotherapie verringert werden.

Der synergistische Effekt der erfindungsgemäßen Kombination wird vorzugsweise beobachtet, wenn die erfindungsgemäße Kombination 0,1 bis 20 mg/kg, insbesondere 0,5 bis 5 mg/kg Wirkstoff der Komponente A (berechnet als o-Acetylsalicylsäure), 0,001 bis 10 mg/kg, insbesondere 0,005 bis 5 mg/kg Wirkstoff der Komponente B jeweils bezogen auf kg Körpergewicht des Patienten bei oraler Applikation enthält.

Des weiteren wird der synergistische Effekt der erfindungsgemäßen Kombination vorzugsweise beobachtet, wenn die erfindungsgemäße Kombination als Komponente A das Salz von o-Acetylsalicylsäure mit Lysin in einer Dosierung von 25 bis 500 mg, vorzugsweise in einer Dosierung von 50 bis 350 mg; besonders bevorzugt in einer Dosierung von 75 bis 200 mg (berechnet als o-Acetylsalicylsäure) und als Komponente B Atorvastatin in einer Dosierung von 1 bis 200 mg, vorzugsweise in einer Dosierung von 5 bis 80 mg, besonders bevorzugt in einer Dosierung von 20 bis 40 mg, Simvastatin in einer Dosierung von 2,5 bis 160 mg, vorzugsweise in einer Dosierung von 5 bis 80 mg, besonders bevorzugt in einer Dosierung von 10 bis 40 mg, Pravastatin in einer Dosierung von 2 bis 100 mg, vorzugsweise in einer Dosierung von 5 bis 80 mg, besonders bevorzugt in einer Dosierung von 20 bis 40 mg, oder Lovastatin in einer Dosierung von 5 bis 200 mg, vorzugsweise in einer Dosierung von 5 bis 80 mg, besonders bevorzugt in einer Dosierung von 10 bis 40 mg enthält.

Der synergistische Effekt der erfindungsgemäßen Kombinationen wird vorzugsweise beobachtet, wenn die Komponenten A und B der erfindungsgemäßen Kombinationen in einem Verhältnis von 2 : 1 bis 100 : 1, bevorzugt 2 : 1 bis 10 : 1, besonders bevorzugt 2,5 : 1 bis 5 : 1 bezüglich A (berechnet als o-Acetylsalicylsäure) und B vorliegen.

Unter "Verhältnis" im Sinne der Erfindung wird das Gewichtsverhältnis der einzelnen Komponenten verstanden.

Gegebenenfalls kann es erforderlich sein, von den genannten Mengen abzuweichen, und zwar in Abhängigkeit vom Körpergewicht bzw. der Art des Applikationsweges, vom individuellen Verhalten gegenüber den Medikamenten, der Art von deren Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die erfindungsgemäße Kombination zeichnet sich weiterhin durch eine überraschend gute Verträglichkeit aus.

Als Komponente A kann des weiteren auch das Salz von o-Acetylsalicylsäure mit einer basischen Aminosäure, bevorzugt Lysin, eingesetzt werden, das bei einer mit einem Malvern 2600D-Gerät unter Standardbedingungen gemessenen Korngrößenverteilung eine mittlere Korngröße oberhalb einer Korngröße von 160 µm und einen Anteil von mehr als 60 % der Teilchen mit einer Korngröße in einem Bereich von 100 bis 200 µm, bevorzugt eine mittlere Korngröße oberhalb einer Korngröße von 170 µm und einen Anteil von mehr als 70 % der Teilchen mit einer Korngröße in einem Bereich von 100 bis 200 µm aufweist. Komponente A weist üblicherweise einen Restfeuchtegehalt von weniger als 0,4 %, vorzugsweise von weniger als 0,3 % und insbesondere von weniger als 0,15 % Wasser auf. Der geringe Restfeuchtegehalt führt zu einer verbesserten Stabilität der Komponente A und folglich auch der erfindungsgemäßen Kombination und des Arzneimittels enthaltend diese Kombination. Das Herstellverfahren ist in der WO 02/005782 beschrieben.

Eine weitere Ausführungsform von Komponente A ist ein Wirkstoffkomplex aus einem Salz der o-Acetylsalicylsäure mit einer basischen Aminosäure, bevorzugt Lysin, und Glycin. Der Wirkstoffkomplex und dessen Herstellung ist in der deutschen Patentanmeldung DE 102005025283 beschrieben. Der Gehalt an Glycin im Wirkstoffkomplex beträgt 8 bis 12, bevorzugt 9 bis 11, besonders bevorzugt 10 Gewichtsprozente bezogen auf den Wirkstoffkomplex. Die vorteilhaften Eigenschaften der vorliegenden Erfindung werden unabhängig von der Korngröße des Wirkstoffkomplexes beobachtet. Die Korngrößenverteilung des Wirkstoffkomplexes weist beispielsweise eine mittlere Korngröße von bevorzugt unterhalb 100 µm, besonders bevorzugt von unterhalb 70 µm auf.

Der Wirkstoffkomplex aus einem Salz der o-Acetylsalicylsäure mit einer basischen Aminosäure und Glycin ist in der deutschen Anmeldung DE 102005025283 beschrieben und kann wie folgt hergestellt werden:

o-Acetylsalicylsäure und die entsprechende Aminosäure werden bei einer Temperatur kleiner gleich 40°C, vorzugsweise von 20 bis 35°C, unter Normaldruck möglichst rasch, bevorzugt in weniger als 20 Minuten, zusammengegeben und zu einer homogenen Phase vermischt, so daß die Temperatur 40°C nicht übersteigt. Zu der so hergestellten homogenen Mischung werden gegebenenfalls Impfkristalle zugesetzt, auf -5 bis 10°C, bevorzugt auf 0 bis 5°C abgekühlt und bei dieser Temperatur 2 bis 8 Stunden, bevorzugt 3 bis 5 Stunden gerührt. Es wird mit gekühltem Aceton und mit der benötigten Menge an gegebenenfalls gekühltem Glycin versetzt. Die Suspension sollte zur Vervollständigung der Kristallisation mindestens eine Stunde unter den vorstehend angegebenen Bedingungen gehalten werden. Erfindungsgemäß bevorzugt ist eine Kristallisationsdauer von 1 bis 10 Stunden unter den vorstehend angegebenen Bedingungen, wobei ein Zeitraum von 1 bis 8 Stunden besonders bevorzugt ist. Es ist gemäß der vorliegenden Erfindung sehr wichtig, daß die Temperatur während des Kristallisationsvorgangs in möglichst engen Grenzen gehalten wird. Die Temperatur darf 5°C nicht übersteigen und sollte vorzugsweise unterhalb von 3°C, besonders bevorzugt zwischen 0 und 2°C, gehalten werden. Als Impfkristalle können Kristalle des gewünschten Produkts verwendet werden. Die Kristallisation erfolgt bevorzugt unter Normaldruck.

Das Kristallisat wird anschließend auf herkömmliche Weise isoliert, beispielsweise durch Filtrieren oder Zentrifugieren. Der Feststoff wird mehrfach mit organischen Lösungsmitteln gewaschen, wobei erfindungsgemäß Alkohole wie beispielsweise Ethanol und/oder Ketone wie Aceton oder Mischungen von Alkoholen und/oder Ketonen, beispielsweise Mischungen von Ethanol und Aceton, oder die Verwendung verschiedener derartiger Lösungsmittel bevorzugt ist.

Der Feststoff wird anschließend unter vermindertem Druck getrocknet. Hierbei sollte die Temperatur unter 50°C, vorzugsweise unter 40°C und besonders bevorzugt unter 35°C gehalten werden. Es sollte ein Druck von weniger als 100 mbar, vorzugsweise von weniger als 50 mbar an den Feststoff angelegt werden. Die Trocknung kann unter herkömmlichen Bedingungen erfolgen, beispielsweise in einem Trockengerät.

Als Lösungsmittel für die Reaktionspartner kommen Wasser beziehungsweise mit Wasser mischbare organische Lösungsmittel wie beispielsweise Alkohole wie Methanol, Ethanol oder isoPropanol, insbesondere Ethanol, Ether wie Tetrahydrofuran (THF) oder Ketone wie Aceton, oder Gemische der genannten Lösungsmittel in Frage. Bevorzugt sind Wasser, Ethanol oder ein Gemisch aus beiden.

Bevorzugt wird die o-Acetylsalicylsäure in Ethanol gelöst und die Aminosäure, bevorzugt Lysin, besonders bevorzugt D,L-Lysin Monohydrat in Wasser gelöst zusammengegeben.

Die Reaktionspartner werden in solchen Mengen eingesetzt, daß die basische Aminosäure in leichtem Überschuss bezogen auf die Mol o-Acetylsalicylsäure vorliegt. Erfindungsgemäß bevorzugt ist ein Molverhältnis von o-Acetylsalicylsäure zu Aminosäure von 1:1,05 bis 1:1,5, wobei ein Verhältnis von o-Acetylsalicylsäure zu Aminosäure von 1:1,05 bis 1:1,2 besonders bevorzugt ist.

Gemäß der vorliegenden Erfindung sollte die o-Acetylsalicylsäurelösung einen Gehalt von 1 bis 10 Gew.-%, vorzugsweise 5 bis 10 Gew.-% und insbesondere bevorzugt von 6 bis 8 Gew.-% o-Acetylsalicylsäure aufweisen. Die Lösung der basischen Aminosäure sollte einen Gehalt von 10 bis 40 Gew.-%, vorzugsweise 15 bis 35 Gew.-% und insbesondere bevorzugt von 20 bis 30 Gew.-% Aminosäure aufweisen.

Das Glycin kann gemäß der vorliegenden Erfindung als Lösung in Wasser oder einem mit Wasser mischbaren organischen Lösungsmittel, wobei als organische Lösungsmittel die vorstehend beschriebenen Lösungsmittel verwendet werden können, der Reaktionsmischung aus den Reaktionspartnern zugegeben werden.

Das Glycin kann gemäß der vorliegenden Erfindung aber auch in Form einer Suspension zugegeben werden. Die Glycin-Suspension kann auf herkömmliche Weise hergestellt werden. Erfindungsgemäß bevorzugt ist die Herstellung einer Glycin-Suspension aus einem Lösungsmittelgemisch aus Wasser und einem Alkohol wie beispielsweise Ethanol.

Ebenso wichtig ist beim erfindungsgemäßen Verfahren das Einhalten einer bestimmten Rührenergie während der Kristallisation. Die homogene Mischung der Ausgangsprodukte darf nur sanft gerührt werden. Die anzulegende Rührenergie sollte nicht größer als 0,1 W pro Liter Reaktionsmedium sein. Erfindungsgemäß bevorzugt ist eine angelegte Rührenergie von 0,04 bis 0,06 W pro Liter Reaktionsmedium. Als Rührer kommen alle herkömmlichen entsprechend regulierbaren Rührgeräte wie beispielsweise ein Rührwerkbehälter mit Stromstörer in Betracht.

Die erfindungsgemäße Kombination wird bevorzugt in der Humanmedizin eingesetzt, eignet sich jedoch auch für die Veterinärmedizin, insbesondere zur Behandlung von Säugetieren.

### Ausfuhrungsbeispiele:

Die vorliegende Erfindung wird nachstehend anhand von nicht einschränkenden bevorzugten Beispielen (1kg-Maßstab; Dosiseinheit 450mg) näher dargestellt. Soweit nicht anderweitig angegeben, beziehen sich alle Mengenangaben auf Gewichtsprozent und Verhältnisse auf Gewichtsverhältnisse.

### Beispiel 1:

402,9 g D,L-Lysin-o-Acetylsalicylat werden mit 68,4 g Mannitol, 141,1 g mikrokristalliner Cellulose, 112,9 g Natriumcarboxymethylcellulose, 12,0 g hochdisperses Siliciumdioxid und 24,2 g Magnesiumstearat gemischt und mittels Trockengranulation kompaktiert. Die entstehenden Briketts werden in 2 Stufen gesiebt.

106,7 g mikrokristalline Cellulose, 48,9 g Mannitol, 7,8 g Natriumcarboxymethylcellulose, 44,4 g mikronisiertes Simvastatin, 6,7 g Hydroxypropylmethylcellulose, 1,1 g Natrium-laurylsulfat und 3,3 g Ascorbinsäure werden feucht granuliert und das entstandene Granulat einmal gesiebt.

Die beiden hergestellten Granulate werden vereinigt, homogenisiert und mit 19,6 g Magnesiumstearat nachgemischt. Es erfolgt eine direkte Abfüllung des Granulates oder eine Verpressung zu Tabletten. Ein Überziehen der Arzneiform kann optional erfolgen.

### Beispiel 2:

444,4 g Wirkstoffkomplex D,L-Lysin-o-Acetylsalicylat mit Glycin aus Beispiel 5 und 44,4 g mikronisiertes Simvastatin werden mit 90,0 g Mannitol, 244,4 g mikrokristalliner Cellulose, 120,7 g Natriumcarboxymethyl-cellulose, 12,2 g hochdisperses Siliciumdioxid und 24,2 g Magnesiumstearat gemischt und mittels Trockengranulation kompaktiert. Die entstehenden Briketts werden in 2 Stufen gesiebt. Nach Nachmischung des Granulates mit 19,6 g Magnesiumstearat erfolgt eine direkte Abfüllung des Granulates oder eine Verpressung zu Tabletten. Ein Überziehen der Arzneiform kann optional erfolgen.

### Beispiel 3:

402,9 g D,L-Lysin-o-Acetylsalicylat werden mit 149,9 g mikrokristalliner Cellulose, 68,4 g Mannitol, 112,9 g Natriumcarboxymethylcellulose gemischt.

44,4 g mikronisiertes Simvastatin, 44,4 g Mannit und 133,2 g Hydroxypropylcellulose werden gemischt, extrudiert und das erhaltene Extrudat zerkleinert (Korngröße <0,315 [mm]).

Die Pulvermischung mit D,L-Lysin-o-Acetylsalicylat wird mit dem Simvastatinextrudatgranulat homogenisiert, im Anschluss mit 43,8 g Magnesiumstearat nachgemischt und direkt abgefüllt oder zu Tabletten verpresst. Ein Überziehen der Arzneiform kann optional erfolgen.

### Beispiel 4:

Wie Beispiel 1, aber nach Verpressen des Granulates zu Tabletten erfolgt eine Lackierung der Tabletten mit einer wäßrigen Opadry II-Suspension (Nummer 85G), einem Feuchteschutzlack auf der Basis von PVA. Zur Lackierung werden 30,0g Opadry II (Nummer 85G) benötigt.

### Beispiel 5: Wirkstoffkomplex aus D,L-Lysin-o-Acetylsalicylat mit 10 % Glycin

In einen sterilen und pyrogenfreien Rührwerkbehälter mit Stromstörer gibt man über einen Sterilfilter eine pyrogenfreie Lösung von 40,0 kg o-Acetylsalicylsäure in 500 kg Ethanol. Bei 20 bis 30°C fügt man unter Rühren und Kühlen innerhalb kurzer Zeit (unter 15 Minuten) eine sterilfiltrierte und pyrogenfreie Lösung von 36,4 kg D,L-Lysin Monohydrat in 110 kg pyrogenfreiem Wasser hinzu, so dass eine Temperatur von 35°C nicht überschritten wird. Es werden mindestens 20 g an sterilen Impfkristallen hinzugefügt und die bereits kristallisierende Mischung unter reduzierter Rührerdrehzahl auf 2°C gekühlt. Danach werden 490 kg pyrogenfreies und temperiertes Aceton und eine aseptisch und temperierte vorbereitete Suspension von 8,0 kg Glycin in 25,0 kg pyrogenfreiem Wasser und 90 kg Ethanol hinzugefügt. Unter weiterem Kühlen bei 2°C wird die Suspension 1 bis 8 Stunden weiter gerührt. Erst dann wird das Kristallgemisch unter aseptischen Bedingungen auf einem Filter oder einer Zentrifuge isoliert. Das Feuchtprodukt wird auf dem Trennapparat mit pyrogenfreiem Ethanol und Aceton gewaschen und unter aseptischen Bedingungen bis zu einem Druck ≤ 50 mbar und einer Temperatur von nicht mehr als 40°C getrocknet. Anschließend wird das Fertigprodukt in Gebinde mit PE-Inlinern abgefüllt und versiegelt. Es werden 60 bis 70 kg (75 bis 87 % d. Theorie) des Titelprodukts mit einer Restfeuchte < 0,3 % mit einer mittleren Korngröße von 41 µm erhalten.

### Schmelzpunktbestimmung von Beispiel 5 durch DSC (Differential Scanning Calorimetry):

Die Schmelzpunktbestimmungen durch DSC werden mit dem Gerät Pyris-1 der Firma PerkinElmer mit einer Heizrate von 20 K/min. durchgeführt. Trockener Stickstoff wird als Schutzgas verwendet. Die charakteristischen DSC-Kurven von Produkt gemäß Beispiel 5 zeigen zwei Peaks, einen endothermischen bei 147,9 ± 1,44°C gefolgt von einem exothermischen bei 153,0 ± 1,0°C. Der endothermische Peak resultiert aus dem Schmelzprozeß, wogegen der exothermische Peak aus einer Überlappung von Zerfall und partieller Kristallisation eines Zerfallprodukts (z.B. o-Acetylsalicylsäure) in der geschmolzenen Phase resultiert.

### Stabilitätsdaten

Die Tabletten werden in Braunglasflaschen mit Trockenmittel im Deckel verpackt und in den Klimakammern bei 25 °C / 60 % relativer Feuche und 40 °C / 75 % relativer Feuchte gelagert.

Die Tabletten werden regelmäßig ausgelagert und auf ihren Gehalt an Acetylsalicylsäure, Salicylsäure als Abbauprodukt von Acetylsalicylsäure, Simvastatin und Hydroxysäure als Abbauprodukt von Simvastatin analysiert. Als Analysenmethode wird eine selektive HPLC-Methode eingesetzt.

Die untersuchten Tabletten werden wie oben beschrieben hergestellt und sind folgendermaßen definiert:
- ASS-SIMVA TABL 100+20MG enthält 100 mg o-Acetylsalicylsäure und 20 mg Simvastatin,
- ASS LYS-SIMVA TABL 100+20MG enthält 181 mg D,L-Lysin-o-Acetylsalicylat (entsprechen 100 mg o-Acetylsalicylsäure) und 20 mg Simvastatin,
- ASS LYS GLY-SIMVA TABL 100+20MG enthält 200 mg Wirkstoffkomplex D,L-Lysin-o-Acetylsalicylat mit Glycin (Beispiel 5) (entsprechen 100 mg o-Acetylsalicylsäure) und 20 mg Simvastatin.

**Tabelle 1: Gehalt an o-Acetylsalicylsäure (mg/Tablette)**

| | Anfang | 3 Monate, 25 °C / 60 % rel. Feuchte | 3 Monate, 40 °C / 75 % rel. Feuchte |
|---|---|---|---|
| ASS - SIMVA TABL 100+20MG | 95 | 93 | 28 |
| ASS LYS - SIMVA TABL 100+20MG | 95 | 94 | 90 |
| ASS LYS GLY - SIMVA TABL 100+20MG | 96 | 95 | 93 |

**Tabelle 2: Salicylsäure (% bezogen auf deklarierten Gehalt Acetylsalicylsäure) als Abbauprodukt von Acetylsalicylsäure**

| Δ: Differenz zum Anfangswert | | | |
|---|---|---|---|
| | Anfang | 3 Monate, 25 °C / 60 % rel. Feuchte | 3 Monate, 40 °C / 75 % rel. Feuchte |
| ASS - SIMVA TABL 100+20MG | 0,9 | 3,0 (Δ 2,1%) | 18,8 (Δ 17,9 %) |
| ASS LYS - SIMVA TABL 100+20MG | 3,3 | 4,6 (Δ 1,3 %) | 8,8 (Δ 5,5 %) |
| ASS LYS GLY - SIMVA TABL 100+20MG | 2 | 2,8 (Δ 0,8 %) | 5,0 (Δ 3,0 %) |

**Tabelle 3: Ergebnisse: Gehalt an Simvastatin (mg/Tablette)**

| | Anfang | 3 Monate, 25 °C / 60 % rel. Feuchte | 3 Monate, 40 °C / 75 % rel. Feuchte |
|---|---|---|---|
| ASS - SIMVA TABL 100+20MG | 23 | 20 | 13 |
| ASS LYS - SIMVA TABL 100+20MG | 21 | 21 | 21 |
| ASS LYS GLY - SIMVA TABL 100+20MG | 22 | 21 | 22 |

**Tabelle 4: Hydroxysäure (% bezogen auf deklarierten Gehalt Simvastatin) als Abbauprodukt von Simvastatin**

| Δ: Differenz zum Anfangswert | | | |
|---|---|---|---|
| | Anfang | 3 Monate, 25 °C / 60 % rel. Feuchte | 3 Monate, 40 °C / 75 % rel. Feuchte |
| ASS - SIMVA TABL 100+20MG | 0,6 | 0,8 (Δ 0,2 %) | 4,4 (Δ 3,8 %) |
| ASS LYS - SIMVA TABL 100+20MG | 0,3 | 0,2 (Δ - 0,1 %) | 0,5 (A 0,3 %) |
| ASS LYS GLY - SIMVA TABL 100+20MG | 0,3 | 0,1 (Δ - 0,2 %) | 0,4 (Δ 0,1 %) |

### Ergebnis:

Die erfindungsgemäßen Tabletten (ASS LYS-SIMVA TABL 100+20MG und ASS LYS GLY-SIMVA TABL 100+20MG) enthaltend eine fixe Kombination aus D,L-Lysin-o-Acetylsalicylat oder dessen Wirkstoffkomplex mit Glycin, und Simvastatin, die zusammen in einer gemeinsamen Matrix in derselben Schicht vorliegen, zeigen eine höhere Stabilität als das Vergleichsbeispiel enthaltend o-Acetylsalicylsäure und Simvastatin (ASS-SIMVA TABL 100+20MG)

## Patentansprüche

1. Fixe Kombination enthaltend ein Salz von o-Acetylsalicylsäure mit einer basischen Aminosäure als Komponente A und einem HMG-CoA-Reduktase-Inhibitor als Komponente B.

2. Kombination gemäß Anspruch 1 mit Simvastatin als HMG-CoA-Reduktase-Inhibitor.

3. Kombination gemäß einem der Ansprüche 1 oder 2, **dadurch gekennzeichnet, dass** die basische Aminosäure Lysin ist.

4. Kombination gemäß einem der Ansprüche 1 bis 3, **dadurch gekennzeichnet, dass** die Komponente A ein Wirkstoffkomplex bestehend aus einem Salz der o-Acetylsalicylsäure mit einer basischen Aminosäure und Glycin ist.

5. Kombination gemäß Anspruch 4, **dadurch gekennzeichnet, dass** der Wirkstoffkomplex zu 8 bis 12 Gewichtsprozenten aus Glycin besteht.

6. Kombination gemäß einem der Ansprüche 4 bis 5, **dadurch gekennzeichnet, dass** der Wirkstoffkomplex zu 10 Gewichtsprozenten aus Glycin besteht und der Schmelzbereich des Wirkstoffkomplexes eine endothermische Peaktemperatur von 148 ± 2°C und eine exothermische Peaktemperatur von 153 ± 2°C aufweist.

7. Arzneimittel, enthaltend eine fixe Kombination gemäß einem der Ansprüche 1 bis 6 und einen oder mehrere weitere geeignete Hilfsstoffe.

8. Arzneimittel gemäß Anspruch 7, **dadurch gekennzeichnet, dass** es sich um eine stabile Darreichungsform handelt, in der Komponente A und B zusammen in einer gemeinsamen Matrix in derselben Schicht vorliegen.

9. Verfahren zur Herstellung eines Arzneimittels gemäß Anspruch 7, **dadurch gekennzeichnet, dass** Komponente A und B gemischt und direkt abgefüllt oder tablettiert werden.

10. Verfahren gemäß Anspruch 9, **dadurch gekennzeichnet, dass** Komponente A und B gemeinsam trocken granuliert werden und im Anschluss direkt abgefüllt oder tablettiert werden

11. Verfahren gemäß Anspruch 9, **dadurch gekennzeichnet, dass** Komponente A trocken granuliert und Komponente B feucht granuliert wird und nach Mischung beider Granulate das daraus resultierende Granulat direkt abgefüllt oder tablettiert wird.

12. Verfahren gemäß Anspruch 9, **dadurch gekennzeichnet, dass** Komponente A in Form einer Pulvermischung und Komponente B als Extrusionsgranulat verwendet werden.

13. Verfahren gemäß Anspruch 9, **dadurch gekennzeichnet, dass** Komponente A und Komponente B als zwei voneinander unabhängige Extrusionsgranulate verwendet werden.

14. Verfahren gemäß Anspruch 9, **dadurch gekennzeichnet, dass** die Komponenten A und B als gemeinsames Extrusionsgranulat verwendet werden.

15. Kombination gemäß einem der Ansprüche 1 bis 6 zur Prävention von Herzkreislauferkrankungen.

16. Kombination gemäß Anspruch 15 zur Prävention von Herzkreislauferkrankungen bei Patienten, die ein erhöhtes Risiko besitzen, an einer Herzkreislauferkrankung zu erkranken.

17. Verwendung der Kombination gemäß einem der Ansprüche 1 bis 6 zur Herstellung eines Arzneimittels zur Prävention von Herzkreislauferkrankungen.

## Claims

1. Fixed combination comprising a salt of o-acetylsalicylic acid with a basic amino acid as component A and an HMG-CoA reductase inhibitor as component B.

2. Combination according to Claim 1 with simvastatin as an HMG-CoA reductase inhibitor.

3. Combination according to one of Claims 1 or 2, **characterized in that** the basic amino acid is lysine.

4. Combination according to one of Claims 1 to 3, **characterized in that** the component A is an active substance complex consisting of a salt of o-acetylsalicylic acid with a basic amino acid and glycine.

5. Combination according to Claim 4, **characterized in that** the active substance complex consists to 8 to 12 percent by weight of glycine.

6. Combination according to one of Claims 4 to 5, **characterized in that** the active substance complex consists to 10 percent by weight of glycine and the melting range of the active substance complex has an endothermic peak temperature of 148 ± 2°C and an exothermic peak temperature of 153 ± 2°C.

7. Medicament comprising a fixed combination according to one of Claims 1 to 6 and one or more further suitable excipients.

8. Medicament according to Claim 7, **characterized in that** it is a stable presentation form, in which component A and B are present together in a joint matrix in the same layer.

9. Process for the production of a medicament according to Claim 7, **characterized in that** component A and B are mixed and directly filled or tabletted.

10. Process according to Claim 9, **characterized in that** component A and B are dry-granulated together and subsequently directly filled or tabletted.

11. Process according to Claim 9, **characterized in that** component A is dry-granulated and component B is wet-granulated and after mixing of both granulates the granules resulting therefrom are directly filled or tabletted.

12. Process according to Claim 9, **characterized in that** component A is used in the form of a powder mixture and component B is used as extrusion granules.

13. Process according to Claim 9, **characterized in that** component A and component B are used as two extrusion granulates independent of one another.

14. Process according to Claim 9, **characterized in that** the components A and B are used as a common extrusion granulate.

15. Combination according to one of Claims 1 to 6 for the prevention of cardiovascular diseases.

16. Combination according to Claim 15 for the prevention of cardiovascular diseases in patients who have an increased risk of developing a cardiovascular disease.

17. Use of the combination according to one of Claims 1 to 6 for the production of a medicament for the prevention of cardiovascular diseases.

## Revendications

1. Association fixe contenant un sel d'acide o-acétylsalicylique avec un acide aminé basique en tant que composant A et un inhibiteur de HMG-CoA-réductase en tant que composant B.

2. Association selon la revendication 1, avec de la simvastatine en tant qu'inhibiteur de HMG-CoA-réductase.

3. Association selon la revendication 1 ou 2, **caractérisée en ce que** l'acide aminé basique est la lysine.

4. Association selon l'une quelconque des revendications 1 à 3, **caractérisée en ce que** le composant A est un complexe de substances actives consistant en un sel de l'acide o-acétylsalicylique avec un acide aminé basique et glycine.

5. Association selon la revendication 4, **caractérisée en ce que** le complexe de substances actives est constitué de glycine à raison de 8 à 12 % en poids.

6. Association selon l'une quelconque des revendications 4 et 5, **caractérisée en ce que** le complexe de substances actives est constitué de glycine à raison de 10 % en poids et le domaine de fusion du complexe de substances actives présente une température endothermique maximale de 148 ± 2 °C et une température exothermique maximale de 153 ± 2 °C.

7. Médicament, contenant une association fixe selon l'une quelconque des revendications 1 à 6 et un ou plusieurs autres adjuvants appropriés.

8. Médicament selon la revendication 7, **caractérisé en ce qu'**il s'agit d'une forme d'administration stable, dans laquelle les composants A et B sont présents ensemble dans la même couche dans une matrice commune.

9. Procédé pour la fabrication d'un médicament selon la revendication 7, **caractérisé en ce qu'**on mélange les composants A et B et on les introduit directement dans un contenant ou les transforme en comprimés.

10. Procédé selon la revendication 9, **caractérisé en ce qu'**on granule à sec les composants A et B ensemble et ensuite on les introduit directement dans un contenant ou les transforme en comprimés.

11. Procédé selon la revendication 9, **caractérisé en ce qu'**on soumet le composé A à une granulation à sec et le composé B à une granulation par voie humide et après mélange des deux granulés on introduit directement dans un contenant le granulé résultant ou on le transforme en comprimés.

12. Procédé selon la revendication 9, **caractérisé en ce qu'**on utilise le composant A sous forme d'un mélange en poudre et le composant B sous forme de granulé extrudé.

13. Procédé selon la revendication 9, **caractérisé en ce qu'**on utilise le composant A et le composant B sous forme de deux granulés extrudés indépendamment l'un de l'autre.

14. Procédé selon la revendication 9, **caractérisé en ce qu'**on utilise les composants A et B sous forme de granulé extrudé commun.

15. Association selon l'une quelconque des revendications 1 à 6, destinée à la prévention de maladies cardiovasculaires.

16. Association selon la revendications 15, destinée à la prévention de maladies cardiovasculaires chez des patients qui présentent un risque élevé de souffrir d'une maladie cardiovasculaire.

17. Utilisation de l'association selon l'une quelconque des revendications 1 à 6, pour la fabrication d'un médicament destiné à la prévention de maladies cardiovasculaires.
